# EUROPEAN PATENT APPLICATION

(11) **EP 3 888 635 A1**
(43) Date of publication of application: **06.10.2021**
(21) Application number: 19908121.7
(22) Date of filing: 07.01.2019
(51) Int. Cl.: A61K 9/113, A61K 9/00, A61K 31/573, A61K 45/06

(54) **DRUG DELIVERY PLATFORM USING W/O/W-TYPE TRIOLEIN EMULSION PROMOTIVE OF BLOOD-BRAIN BARRIER OPENING**

(71) Applicant: Pusan National University Industry-University Cooperation Foundation, Busan 46241 (KR)
(72) Inventor: KIM, Hak Jin, Busan 48515 (KR); KIM, Cheol Min, Busan 48516 (KR); JHUN, Byung Hak, Yangsan-si, Gyeongsangnam-do 50649 (KR); YOO, Jin-Wook, Busan 46241 (KR); LEE, Juho, Busan 47546 (KR)
(74) Representative: Roos, Peter
(86) International application number: PCT/KR2019/000201
(87) International publication number: WO 2020/145415

(57) **Abstract**

The present invention relates to a composition for drug delivery to a tissue having a tight junction comprising a triolein emulsion of an internal water phase/oil phase/external water phase (Water/Oil/Water, W/O/W) structure in which oil droplet comprises triolein and water droplets are enclosed in the oil droplet, as an active ingredient, and a method of preparing the same, and the triolein emulsion of the W/O/W-type structure according to the present invention has excellent safety because it retains the BBB opening activity of triolein as it is and the water droplets are enclosed in the oil droplets and contain surfactants and can further enclose fat-soluble drugs in the oil droplets and thus it can more effectively deliver drugs to tissues having tight junctions such as the brain, testicles, and retina.

## Description

### [Technical Field]

The present invention relates to a composition for drug delivery comprising a triolein emulsion having an internal water phase/oil phase/external water phase structure as an active ingredient and a method of preparing the same.

### [Background Art]

To treat CNS-related diseases, various types of therapeutic agents such as proteins, peptides, nucleosides, nucleotides, antiviral agents, tumor suppressors, antibiotics, and prodrugs, precursors, derivatives, and intermediates thereof, have been developed. In addition, many of the known neuroactive peptides offer additional possibilities as useful therapeutic agents. Since the neuroactive peptides play important biochemical roles in the CNS, for example neurotransmitters and/or neuromodulators, delivery of such various sequences of peptides to the CNS can provide many opportunities for therapeutic benefits. Delivery of endogenous and synthetic opioid peptides such as enkephalin is used to induce analgesia.

However, there are many obstacles to use many compounds as therapeutics for CNS, the most important of which is that the brain has a barrier system. The brain barrier system has two major components: the choroid plexus and the blood vessel-brain barrier (BBB). The choroidal plexus separates cerebrospinal fluid (CSF) and blood, and the BBB separates blood and brain ISF. In particular, the BBB is a gateway that limits the transfer of substances from the blood to the brain tissue, and has high selective permeability and plays a role of protecting harmful substances from moving to the brain. In the BBB, a tight junction is formed between cells and cells, so fat-soluble substances can pass through the BBB, but water-soluble substances or polymeric substances are difficult to pass through the BBB, which is an obstacle to drug delivery for the treatment of brain diseases.

Therefore, in order to overcome this limitation, various technologies for delivering drugs to the brain through the BBB have been developed.

### [Disclosure]

### [Technical Problem]

It is an object of the present invention to provide a composition for drug delivery that can effectively deliver a drug to tissues having a tight junction by enhancing the permeability of the blood vessel-brain barrier.

Another object of the present invention is to provide a method of preparing the composition for drug delivery.

### [Technical Solution]

In order to achieve the above object, the present invention provides a composition for drug delivery to a tissue having a tight junction comprising a triolein emulsion of an internal water phase/oil phase/external water phase (Water/Oil/Water, W/O/W) structure in which oil droplet comprises triolein and water droplets are enclosed in the oil droplet, as an active ingredient.

In order to achieve the above other object, the present invention provides a method of preparing a composition for drug delivery to a tissue having a tight junction comprising: preparing a first triolein emulsion by mixing triolein and water comprising a surfactant; and preparing a W/O/W type triolein emulsion comprising triolein droplets having an average diameter of more than 5 µm and less than 15 µm, by adding water to the first triolein emulsion and treating a power source.

### [Advantageous Effects]

The present invention relates to a composition for drug delivery to a tissue having a tight junction comprising a triolein double emulsion having a structure of water phase/oil phase/water phase (W/O/W) as an active ingredient and it solves the problem that the emulsion is rapidly destroyed due to the large difference in density between water and triolein in conventional triolein emulsion and it is possible to adjust the particle size during manufacture to reduce the size deviation between particles and since it can make stable particles and at the same time exhibits the activity of loosening the tight junction of the BBB of triolein as it is, it can be usefully used as a composition for drug delivery to a tissue having a tight junction, that is, to the brain, retina or testis.

### [Description of Drawings]

FIG. 1 schematically shows a double structure of triolein emulsion according to an embodiment of the present invention.
FIG. 2 shows a result of observing under a microscope a triolein emulsion of W/O/W double structure in which a red pigment is enclosed prepared in an embodiment of the present invention.
FIG. 3 shows a result of observing by a microscope to confirm the average particle diameter of the triolein emulsion of W/O/W double structure prepared in an embodiment of the present invention.
FIG. 4 shows a result of confirming that the triolein emulsion of W/O/W double structure in which trypan blue is enclosed, which is prepared in an embodiment of the present invention, is transmitted through the BBB to the inside of the brain.
FIG. 5 shows an MRI photograph of a brain injected with the triolein emulsion of W/O/W double structure in an embodiment of the present invention.

### [Best Mode]

The inventors of the present invention have recognized that the BBB can open when treating the triolein emulsion to the brain, but the difference in density between water and triolein (lipid) is very large, so the formulation is unstable and there is a limit to practical application in clinical practice and have conducted researches to solve this problem, and prepared a triolein emulsion that has no side effects and is much more stable, thereby completing the present invention.

Accordingly, the present invention provides a composition for drug delivery to a tissue having a tight junction comprising a triolein emulsion of an internal water phase/oil phase/external water phase (Water/Oil/Water, W/O/W) structure in which oil droplet comprises triolein and water droplets are enclosed in the oil droplet, as an active ingredient.

At this time, the tissue having the tight junction may be a brain, retina, or testicular tissue.

Triolein is a representative lipid that causes fat embolism syndrome in clinical practice, and when a certain amount of triolein emulsion of a certain size is injected into the carotid artery, it causes temporary fat embolism in the cerebrovascular vessels and loosens the tight junction, thereby temporarily opening the BBB. Thus the triolein emulsion of the W/O/W type structure according to the present invention can contain a surfactant in the triolein droplet while maintaining such BBB opening activity, thereby improving the stability of the particles, and since water droplets are enclosed inside the triolein droplets and it is possible to minimize the destruction of the formulation due to the difference in density between triolein and water., it can be usefully used as a composition for drug delivery to tissues having tight junctions, especially to the brain.

In addition, since the effect of loosening the tight junction is also shown in the retina or testis tissue including a membrane similar to the BBB, the composition according to the present invention can effectively deliver drugs not only to the brain, but also to the retina or testis.

At this time, the average diameter of the oil droplets may be more than 5 µm and less than 15 µm, preferably, the diameters of all oily droplets may be more than 5 µm and less than 10 µm, and if the average diameter is less than 5 µm, it is not possible to cause a temporary clogging of blood vessel, and if it exceeds 15 µm, it causes severe temporary blockage of blood vessels, which undesirably decreases safety.

In an embodiment of the present invention, a water-soluble drug or a water-soluble pigment may be further included in the internal water phase and it is preferable that by including a water-soluble substance in the internal water phase enclosed in the triolein droplet, the triolein loosens the tight junction of the BBB and penetrates into tissues having tight junctions such as the brain, so that drugs contained in the internal water phase can be effectively delivered to the inside of the tissue.

The drug used in the present invention is a substance capable of inducing a desired biological or pharmacological effect by promoting or inhibiting a physiological function in the body of an animal or human, and it means a chemical or biological substance or compound suitable for administration to an animal or human. It has a preventive effect on organic matter by preventing unwanted biological effects such as infection prevention, alleviates condition such as pain or infection resulting from disease and can play a role of alleviating, or reducing or completely eliminating disease from organic matter.

At this time, the water-soluble drug may be an anticancer agent, an aminoglycoside antibiotic, an antifungal agent, an anti-aging agent or an antibody drug, and more preferably, it may be any one or more selected from the group consisting of doxorubicin, cytarabine, vinblastine, rituximab, trastuzumab, gentamicin and tobramycin, but it is not limited thereto.

In addition, the water-soluble pigment may be any one or more selected from the group consisting of trypan blue, Evans blue, Congo red, and methylene blue, but it is not limited thereto.

In one embodiment of the present invention, the oil phase may further include a fat-soluble drug, and more preferably, the fat-soluble drug may be dexamethasone and thus a drug exhibiting BBB-closing activity such as dexamethasone is further encapsulated so that the triolein opens the BBB and then the drug is delivered to the brain, and the BBB is quickly closed so that the drug can be effectively delivered without side effects, which is preferable.

That is, the composition can deliver the drug to the brain by improving the BBB permeability of the drug by loosening the tight junction of the blood-brain barrier (BBB) by triolein.

In the present invention, the composition for drug delivery may further include one or more pharmaceutically acceptable carriers, excipients or diluents in addition to a pharmaceutically effective amount of a triolein emulsion or drug of W/O/W type structure.

In the present invention, the "pharmaceutically effective amount" means an amount sufficient for the drug to be administered to an animal or human to exhibit a desired physiological or pharmacological activity, and it may be appropriately changed according to the age, weight, health condition, sex, route of administration and treatment period of the subject to be administered.

In addition, the "pharmaceutically acceptable" in the present invention means that it is physiologically acceptable and, when administered to humans, it usually does not cause allergic reactions such as gastrointestinal disorders and dizziness or similar responses.

Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate and mineral oils.

In addition, the composition for drug delivery may further include a filler, an anti-aggregating agent, a lubricant, a wetting agent, a flavoring agent, an emulsifying agent, and a preservative.

The composition for drug delivery according to the present invention may be administered through various routes including oral, transdermal, subcutaneous, intravenous, or intramuscular, and the dosage of the drug can be appropriately selected according to several factors such as the route of administration, the patient's age, sex, weight, and the patient's severity. In addition, the composition for drug delivery of the present invention may be administered in parallel with a known compound capable of enhancing the desired effect of the drug.

In addition, the present invention provides a method of preparing a composition for drug delivery to a tissue having a tight junction comprising: preparing a first triolein emulsion by mixing triolein and water comprising a surfactant; and preparing a W/O/W type triolein emulsion comprising triolein droplets having an average diameter of more than 5 µm and less than 15 µm, by adding water to the first triolein emulsion and treating a power source.

Conventionally, triolein emulsion was prepared by mixing triolein and water several times using a 3-way syringe valve, but since triolein and water are not mixed at all, there was a limit that layer separation occurs very quickly to destruct the emulsion system. Moreover, there is a problem that it is also very difficult to control the particle size of the triolein emulsion in this manner.

On the other hand, according to the present invention, since the average diameter of the particles can be easily adjusted during the manufacturing process, only large amounts of particles having the most effective average diameter can be manufactured, so that even a small amount of triolein can provide a sufficient BBB opening effect and it is possible to prepare a W/O/W-type triolein emulsion capable of effectively delivering drugs to tissues having tight junctions such as the brain, retina or testis. In addition, it can be easily and conveniently manufactured using a commonly used syringe, and reproducibility is also very good.

In this case, the first triolein emulsion may be prepared by mixing 0.2 parts by weight to 0.8 parts by weight of a surfactant and 20 parts by weight to 50 parts by weight of water containing the same based on 100 parts by weight of triolein, but it is not limited thereto.

In one embodiment of the present invention, the water containing the surfactant may further comprise a water-soluble drug or a water-soluble pigment.

At this time, the water-soluble drug may be an anticancer agent, an aminoglycoside antibiotic, an antifungal agent, an anti-aging agent or an antibody drug, and more preferably, it may be any one or more selected from the group consisting of doxorubicin, cytarabine, vinblastine, rituximab, trastuzumab, gentamicin, and tobramycin, but it is not limited thereto.

In addition, the water-soluble pigment may be any one or more selected from the group consisting of trypan blue, Evans blue, Congo red and methylene blue, but it is not limited thereto.

In one embodiment of the present invention, the power source may be selected from the group consisting of a sonictor, a homogenizer and a syringe, but it is not limited thereto.

In one embodiment of the present invention, the triolein droplet may further include a fat-soluble drug, and preferably, the fat-soluble drug may be dexamethasone, but it is not limited thereto.

In addition, preferably, the average diameter of the triolein droplets may be more than 5 µm and less than 10 µm.

Hereinafter, the present invention will be described in more detail through examples. These examples are only intended to illustrate the present invention in more detail, and it will be apparent to those skilled in the art that the scope of the present invention is not limited by these examples according to the gist of the present invention. The examples of the present invention are provided to more completely explain the present invention to those of ordinary skill in the art.

### <Example 1> Preparation of triolein emulsion of W/O/W double structure

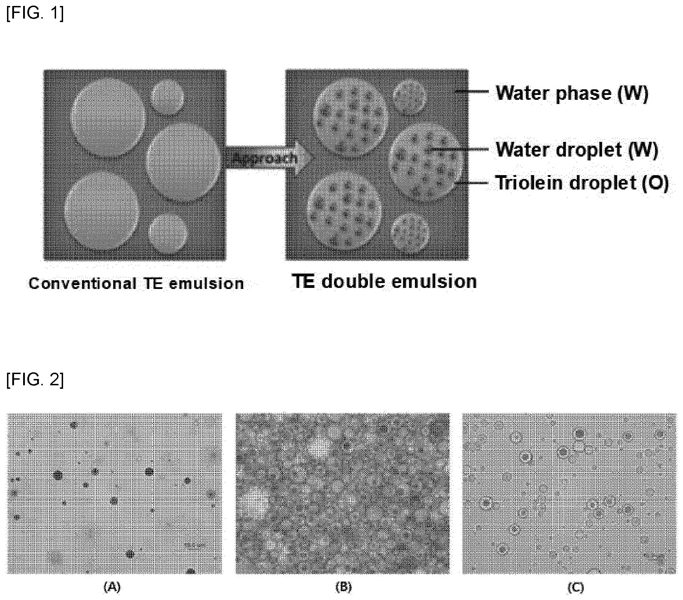

As shown in the schematic diagram of FIG. 1, in order to prepare a triolein emulsion of W/O/W double structure (right) that is more stabilized than the conventional triolein emulsion (left), firstly, a small amount of water containing span 80 as a surfactant and Congo red as a water-soluble pigment and triolein (Sigma Aldrich) were mixed and the red water droplets in the triolein solution was emulsified by sonicator to prepare the first emulsion. At this time, as a result of observing the emulsion using an optical microscope, as shown in FIG. 2A, it was confirmed that the red water-soluble pigment was located inside the triolein in a drop shape.

Thereafter, a certain amount of water containing 1% polyvinyl alcohol was added to the first emulsion, and mixed using a syringe as a power source to prepare a triolein emulsion of W/O/W structure containing triolein droplets with water droplets inside and having an average particle diameter of more than 5 µm and less than 20 µm. At this time, as a result of observing the double emulsion using Congo red, it was as shown in FIG. 2B, and as described above, it was confirmed that most of the water-soluble pigments were enclosed in the triolein droplets.

Thereafter, when used in the experiment, it was dissolved in physiological saline and diluted to a desired concentration, and at this time, the result of observing the emulsion using an optical microscope was as shown in FIG. 2C.

### <Example 2> Measurement of average particle size distribution of triolein emulsion of W/O/W double structure

The triolein emulsion of W/O/W double structure prepared according to the method disclosed in Example 1 above of 200 or more particles were randomly sampled using an optical microscope according to the average particle diameter thereof and the particle diameter was measured and processed statistically by comparing the length with a scale bar using the image J program.

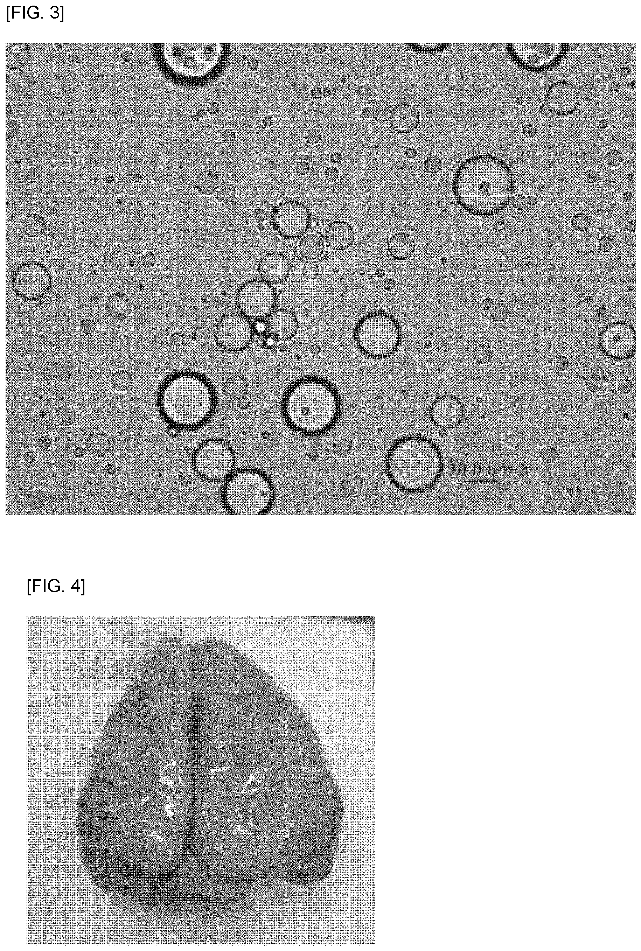

As a result, as shown in FIG. 3 and <Table 1> below, the triolein emulsion mainly exhibited an average particle diameter of 5-20 µm, and the average was about 7.58 µm. At this time, it is estimated that particles having an average particle diameter of 5-20 µm are involved in the temporary BBB opening and when an emulsion containing a large number of particles larger than 20 µm is used, safety may be rather affected.

**[Table 1]**

| | |
|---|---|
| Average | 7.58112 |
| Standard deviation | 3.80486 |

| Particle size distribution (%) | |
|---|---|
| 5 µm and less | 28.1 |
| 5-7.5 µm | 30.8 |
| 7.5-10 µm | 21.2 |
| 10-20 µm | 18.5 |
| At least 20 µm | 1.4 |
| total | 100.0 |

### <Example 3> Confirmation of drug delivery effect of W/O/W double structure triolein emulsion to brain

### (1) Confirmation of BBB opening activity of triolein emulsion

First, a triolein emulsion was prepared according to the method performed in the Example 1, and the average diameter was set to 7 µm, and the concentration of the emulsion was adjusted to 2%. A microcatheter was injected into the femoral artery of a rabbit (1.7 Kg, male) distributed from Semtako, and the tip was placed in one carotid artery, and the emulsion prepared above was injected 7 ml per rabbit. Thereafter, 3 ml of trypan blue was immediately injected into the rabbit, and after 2 hours, the rabbit brain was excised and the blue-stained area was observed. At this time, the principle that when the BBB is opened, the area to be opened is dyed blue was used.

As a result, as shown in FIG. 4, it was found that the area where the BBB of the right brain tissue was opened was stained blue with trypan blue by the triolein emulsion. The blue-stained part of the left brain was confirmed to be due to the lateral circulation of blood vessels.

### (2) Confirmation of drug delivery activity to brain

A triolein emulsion having a W/O/W double structure and an average particle diameter of 7 µm was prepared in the same manner as described above. After diluting by mixing 0.2 ml (2%) of the emulsion in 10 ml of physiological saline, 7 ml of the emulsion diluted in the same procedure as in the experiment in which the trypan blue was added to 1.7 kg male rabbits distributed from Semtako and doxorubicin, a water-soluble drug, were injected, and 2 hours later, the brain of the rabbit was excised and the concentration of doxorubicin was measured using a fluorometer. At this time, three brain tissues were taken off and measured three times.

As a result, as shown in Table 2 below, it was confirmed that the concentration of doxorubicin in the right brain was contained more than an average of 6.12 times the concentration of doxorubicin in the left brain.

**[Table 2]**

| | Doxorubicin concentration in the right brain (RFU*) | Doxorubicin concentration in the left brain (RFU) | Right brain doxorubicin/left brain doxorubicin ratio |
|---|---|---|---|
| | 437 | 78 | 5.60 |
| | 428 | 53 | 8.08 |
| | 428 | 88 | 4.86 |
| | 420 | 64 | 6.56 |
| | 445 | 77 | 5.78 |
| | 431 | 89 | 4.84 |
| | 444 | 78 | 5.69 |
| | 455 | 65 | 7.00 |
| | 464 | 70 | 6.63 |
| Average | 439.11 | 73.56 | 6.12 |
| Standard Deviation | 14.20 | 11.72 | 1.05 |

| | | | |
|---|---|---|---|
| (* : relative fluorescence unit) | | | |

### (3) Confirmation of BBB opening of triolein emulsion through brain magnetic resonance imaging

A triolein emulsion having a W/O/W double structure and an average particle diameter of 7 µm, in which a contrast agent was encapsulated was prepared in the same manner as described above. After diluting by mixing 0.2 ml (2%) of the emulsion in 10 ml of physiological saline, 7 ml of the diluted emulsion was injected into a 1.7 kg male rabbit distributed from Samtaco, and 2 hours later, contrast enhanced magnetic resonance imaging was performed on the brain of the rabbit. This is a photographing method using the property of a contrast agent that penetrates into the brain tissue when the BBB of the brain is opened, and the contrast agent that has penetrated into the brain tissue appears white in the contrast-enhanced magnetic resonance image.

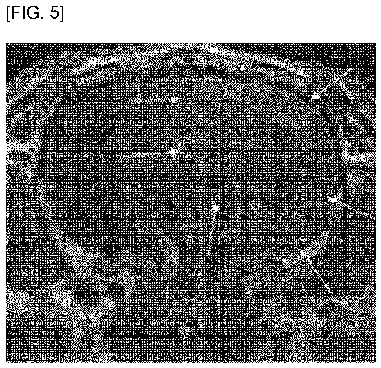

As a result, as shown in FIG. 5, it was found that the left-brain tissue was enhanced with whiter contrast compared to the right brain due to the BBB opened by the triolein emulsion (arrow).

Therefore, when the above experimental results are summarized, the triolein emulsion of W/O/W double droplet structure in which water droplets are enclosed shows the activity of opening the BBB as it is by loosening the tight junction of triolein and can be prepared in a stable formulation and thus it is possible to efficiently perform drug delivery to a tissue having a tight junction, that is, to the brain, retina or testis.

While the present invention has been particularly described with reference to specific embodiments thereof, it is apparent that this specific description is only a preferred embodiment and that the scope of the present invention is not limited thereby to those skilled in the art. That is, the practical scope of the present invention is defined by the appended claims and their equivalents.

## Claims

1. A composition for drug delivery to a tissue having a tight junction comprising a triolein emulsion of an internal water phase/oil phase/external water phase (Water/Oil/Water, W/O/W) structure in which oil droplet comprises triolein and water droplets are enclosed in the oil droplet, as an active ingredient.

2. The composition for drug delivery to a tissue having a tight junction of claim 1, wherein the tissue having a tight junction is brain, retina or testicular tissue.

3. The composition for drug delivery to a tissue having a tight junction of claim 1, wherein the oil droplets have an average diameter of more than 5 µm and less than 15 µm.

4. The composition for drug delivery to a tissue having a tight junction of claim 1, further comprising a water-soluble drug or a water-soluble pigment in the internal water phase.

5. The composition for drug delivery to a tissue having a tight junction of claim 1, further comprising a fat-soluble drug in the oil phase.

6. The composition for drug delivery to a tissue having a tight junction of claim 5, wherein the fat-soluble drug is dexamethasone.

7. The composition for drug delivery to a tissue having a tight junction of claim 1, wherein the composition delivers drug to brain by loosening the tight junction of blood-brain barrier (BBB) by the triolein to improve BBB permeability of the drug.

8. A method of preparing a composition for drug delivery to a tissue having a tight junction comprising:
preparing a first triolein emulsion by mixing triolein and water comprising a surfactant; and
preparing a W/O/W type triolein emulsion comprising triolein droplets having an average diameter of more than 5 µm and less than 15 µm, by adding water to the first triolein emulsion and treating a power source.

9. The method of preparing a composition for drug delivery to a tissue having a tight junction of claim 8, wherein the first triolein emulsion is prepared by mixing 0.2 to 0.8 parts by weight of a surfactant and 20 to 50 parts by weight of water containing the surfactant, based on 100 parts by weight of triolein.

10. The method of preparing a composition for drug delivery to a tissue having a tight junction of claim 8, wherein the water comprising the surfactant further comprises a water-soluble drug or a water-soluble pigment.

11. The method of preparing a composition for drug delivery to a tissue having a tight junction of claim 8, wherein the power source is selected from the group consisting of a sonicator, a homogenizer and a syringe.

12. The method of preparing a composition for drug delivery to a tissue having a tight junction of claim 8, wherein the triolein droplet further comprises a fat-soluble drug.
